# EUROPEAN PATENT APPLICATION

(11) **EP 2 153 880 A1**
(43) Date of publication of application: **17.02.2010**
(21) Application number: 08013743.3
(22) Date of filing: 31.07.2008
(51) Int. Cl.: B01D 53/79, B01D 53/90, C07C 273/16

(54) **Process and plant for the production of a urea solution for use in SCR process for reduction of Nox**

(71) Applicant: UREA CASALE S.A., 6900 Lugano-Besso (CH)
(72) Inventor: Zardi, Federico, 6932 Breganzona (CH)
(74) Representative: Zardi, Marco

(57) **Abstract**

A process and a plant for the preparation of an acqueous solution of urea suitable for use in a SCR process for nitrogen oxides removal, wherein the urea solution from the recovery section of a urea plant is subject to at least one step of evaporation, separating a vapour stream containing water and ammonia, and obtaining a concentrated and substantially ammonia-free solution, and said concentrated solution is diluted to the concentration of urea suitable for use in the SCR process.

## Description

### Field of the invention

The present invention refers to a process and plant for the production of an aqueous solution of urea suitable for use in a SCR process for reduction of nitrogen oxides, namely for removing nitrogen oxides contained in a gaseous stream.

### Prior Art

Selective catalytic reduction or SCR is a known process for removing the nitrogen oxides from a gaseous stream, generated e.g. from the combustion of a fossil fuel. SCR can be used for example for removing nitrogen oxides from the exhaust of a vehicle.

Basically, the SCR converts the nitrogen oxides such as NO and NO₂ into environmentally inert compounds such as nitrogen (N₂) and steam. The optimal temperature range for the SCR process is usually 180 to 350 °C. The conversion requires some ammonia (NH₃), which is provided by injecting an acqueous solution of urea into the gaseous stream, so that the ammonia is generated from in situ decomposition of the urea. Use of this solution avoids the risks and drawbacks of transportation and storage of pure ammonia or a solution thereof. In this specification, the term SCR solution will be used to indicate an acqueous solution of urea suitable for use in the SCR process.

The SCR solution has usually a concentration of 30 to 35 wt% (by weight) of urea. Preferred concentration is about 32 wt%. Production by dissolving the commercially available solid urea into water is not attractive from economical point of view, due to the cost of urea and possible content of additives, such as formaldehyde. Hence, alternative methods have been proposed in the prior art.

WO 2006/096048 discloses a process for the preparation of a urea-comprising acqueous stream suitable for use in a unit for the NOₓ reduction in exhaust gases, wherein the urea-comprising aqueous stream is separated directly from or after a recovery section in a urea production process and thereafter diluted with water until the urea-comprising stream comprises 30-35 wt% urea.

According to well known art, the urea production process takes place in a synthesis section where ammonia and carbon dioxide are reacted under high pressure, producing an acqueous solution containing urea, ammonium carbamate and free ammonia; the solution is sent to a recovery section comprising a plurality of equipments operating at medium and/or low pressure, where the carbamate is dissociated by heating and/or stripping the solution, in order to recycle ammonia and carbon dioxide to the synthesis section. Downstream the recovery section, the urea solution is usually around 70 wt% urea, with a low percentage of residual ammonia. This solution is then concentrated by evaporation, to produce pure urea melt.

In practice, however, an acqueous solution of urea obtained by mere dilution of the output of the recovery section of a state-of-the-art urea plant would not meet the quality requirements of an SCR solution, especially in terms of the ammonia content.

Ammonia content is required to be less than 2000 ppm, but in practice it is necessary to keep the ammonia content below olfactory values, which means a very low concentration, preferably 200 to 500 ppm and more preferably less than 200 ppm. The solution taken from the recovery section, in a state-of-the-art urea plant, has a free ammonia content low but not negligible, around 1-2% (10000 - 20000 ppm) at 65-70 wt% urea. Dilution with water up to 30 - 35 wt% urea, hence, would not allow to meet the above requirement of ammonia <2000 ppm and preferably <200 ppm.

The problem is felt especially, but non exclusively, when the SCR solution is destined to the treatment of the exhaust gases of vehicles, such as heavy vehicles with a diesel engine. In this case, the SCR solution may be handled by non-skilled people, stored in a tank of the vehicle for long periods, exposed to hot summer temperatures, etc...; under these conditions, the risk of release of ammonia vapours must clearly be avoided.

### Summary of invention

The technical problem underlying the invention is to find a suitable and cost-effective way to obtain an aqueous solution of urea suitable for use as additive in a SCR unit for nitrogen oxides removal, with an acceptable ammonia content, by use of the urea-containing acqueous stream produced in the recovery section of a plant for the production of urea.

The problem is solved with a process for the preparation of an acqueous solution of urea suitable for use in a SCR process for nitrogen oxides removal, wherein an acqueous stream containing urea and obtained in the recovery section of a urea production plant is used for preparing said solution, the process being **characterized in that**:
a) said acqueous stream is subject to at least one process step of evaporation, separating a vapour stream containing water and ammonia, and obtaining a concentrated and substantially ammonia-free solution;
b) said concentrated solution is then diluted to obtain a solution with a concentration of urea suitable for use in the SCR process.

The concentration of the SCR solution is preferably 15 to 35 wt% urea and more preferably around 30 to 35 wt%.

The term ammonia-free is used with reference to a very low content of ammonia, so that the ammonia content of the diluted solution meets the requirements for SCR solutions and preferably is below olfactive value. More preferably, the NH3 content of the solution obtained at the above point a) is such that after dilution to 30-35 wt% urea, the NH₃ concentration is less than 2000 ppm, more preferably 200 to 500 ppm and even more preferably <200 ppm.

The evaporation preferably comprises a heating phase and a subsequent separation under vacuum, where the vapour stream containing water and ammonia is separated and the concentrated solution is obtained in liquid phase. Preferably, said separation under vacuum is carried out at a pressure of 0.2 - 0.4 bar (absolute) and more preferably at said pressure and about 110 °C.

According to embodiments of the inventions, the full acqueous stream produced in the recovery section, or only a portion thereof, can be used for the purpose of producing the SCR solution.

The input stream of said evaporation phase may be the acqueous stream as produced in the recovery section, or a stream obtained by subjecting the acqueous stream from the recovery section to a further process step, for removing part of the ammonia. According to a preferred aspect of the invention, there are two evaporation steps in series, wherein the first evaporation obtains a concentrated solution and at least part of said solution is further evaporated in a second evaporation step, obtaining a more concentrated and substantially ammonia-free solution.

Accordingly, and in a preferred embodiment, an acqueous stream from the recovery section of an urea process is subject to a first evaporation step, obtaining a vapour phase containing water and ammonia and a concentrated urea solution in liquid phase; at least a portion of said concentrated urea solution is then subject to a second evaporation step, separating a vapour phase containing water and ammonia and obtaining a further concentrated and substantially ammonia-free urea solution; said further concentrated urea solution is then diluted with water to a predetermined concentration of urea.

This aspect of the invention provides a particularly effective two-steps removal of the ammonia.

Evaporation can be carried out in conventional heat exchanger(s), preferably in shell-and-tube indirect heat exchanger(s), where the urea solution is fed to the tube side. Preferably, steam-heated tube heat exchanger(s) are used, where the urea solution is fed to the tube or plate side, and steam is condensed on the shell side acting as a heat source.

An object of the invention is also a plant adapted to produce a SCR solution according to the above process. The same plant may also be used to obtain other products, such as urea.

In particular, an object of the invention is a plant comprising at least: a synthesis section for converting ammonia and carbon dioxide into a first acqueous stream containing urea, ammonium carbamate and unreacted free ammonia; a recovery section suitable to dissociate the carbamate and recycle the ammonia and carbon dioxide to the synthesis section, and producing a second acqueous stream substantially containing urea, water and residual ammonia, the plant being characterized by comprising a further section suitable to convert at least part of said second acqueous stream into a solution with a lower concentration of urea, for use in SCR process, said further section comprising:
- at least one evaporation unit, adapted to obtain a more concentrated and substantially ammonia-free urea solution in liquid phase,
- a mixing device adapted to dilute said concentrated solution with water.

According to an aspect of the invention, said further section comprises two evaporation units operating in series, wherein the first evaporation unit obtains a concentrated solution and at least part of said solution is sent to the second evaporation unit.

In a preferred embodiment of the invention, there is provided a first evaporation unit, obtaining a vapour phase containing water and ammonia and a concentrated urea solution in liquid phase, and a second evaporation unit, said second unit being fed with at least a portion of said concentrated urea solution from the first unit, and obtaining a further concentrated and substantially ammonia-free urea solution. The first unit preferably comprises an evaporator and a flash vessel; the second unit preferably comprises an evaporator and a vacuum separator. The mixing device is fed with the concentrated and ammonia-free solution obtained in the second unit, and water is added until a suitable concentration of urea is reached.

A further object of the invention is a method for modifying an existing urea plant, in order to operate with the above process.

Hence, an object of the invention is a method for modifying an existing urea production plant comprising a synthesis section, a recovery section and a concentration section, the recovery section obtaining an acqueous stream substantially containing urea, water and residual ammonia, fed to the concentration section for the production of high-purity urea, the method comprising the provision of at least a further section adapted to convert at least a part of said acqueous stream into a solution with a lower concentration of urea, for use in a SCR process, said further section comprising at least one evaporation unit, adapted to obtain a concentrated and substantially ammonia-free urea solution, and a mixing device adapted to dilute said concentrated solution with water.

The main advantage of the invention is the effective removal of ammonia from the urea solution taken from the recovery section of the urea plant, so that the diluted solution can meet the strict requirements for use as additive in SCR systems, especially in vehicles.

It should be noted that the invention provides that the solution taken from the recovery section is first evaporated and concentrated, removing free NH₃ and water, and then diluted with water. This process, rather than the mere dilution (e.g from 70 to 32 wt%), has been found adapted to meet the quality requirements of SCR solutions, and to yield a high-quality solution with residual NH₃ below the olfactive level. The process is also attractive from the economical point of view, avoiding the more expensive process of dissolving solid urea.

Further features and advantages of the invention will be clear from the following description of a preferred embodiment thereof, given as indicative and nonlimiting with reference to the attached drawings.

### Brief description of the drawings

Fig. 1 is a simplified scheme of a plant suitable for the production of SCR solution according to the process of the invention.
Fig. 2 is a scheme of a preferred embodiment of the process of invention.
Fig. 3 is more detailed example of a preferred implementation of the scheme of Fig. 2.

### Detailed description of a preferred embodiment

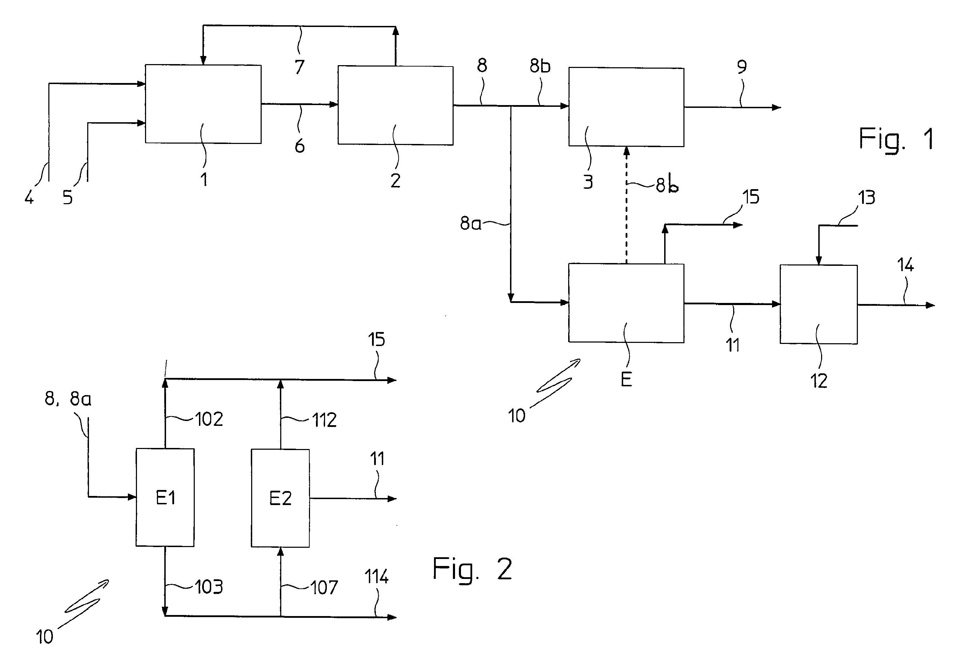

Fig. 1 shows a general block diagram of a plant for the production of urea. The plant comprises basically a high-pressure (HP) synthesis section 1, a recovery section 2 and a concentration section 3. The HP synthesis section 1 converts an ammonia input 4 and carbon dioxide input 5 into an acqueous stream 6, containing urea, ammonium carbamate (NH₃)₂CO₂ and free ammonia; the recovery section comprises medium and/or low-pressure equipments adapted to dissociate the carbamate and then recycle ammonia and carbon dioxide to the synthesis section 1 via the stream 7, and producing an acqueous stream 8 substantially containing urea, water and residual free ammonia; the concentration section 3 comprises equipments for removing water and obtaining urea melt 9 of high-purity, e.g. 99% or more, depending on the use.

All urea plants follow the basic scheme of Fig. 1; the equipments of sections 1, 2 and 3 are not described in detail as they are well known in the art.

Typically, stream 8 contains around 65-70% urea and around 30% water, with a residual 1-2% free ammonia. According to the invention, at least a portion of said acqueous stream 8 is fed to a further section 10 to produce an SCR solution 14, comprising 30-35% urea and adapted for use in SCR process for NOx reduction.

Referring to Fig. 1, a first portion 8a of the stream 8 is used to produce the SCR solution 14, while the remaining second portion 8b is sent to the concentration section 3. Said first portion 8a is fed to an evaporation and vacuum separation unit E, where a vapour phase 15 containing water and ammonia is separated, and a concentrated, ammonia-free solution 11 is obtained in the liquid phase. This solution 11 is fed to a mixer 12 it is diluted with water 13, up to a suitable concentration of urea which is preferably 30 to 35 wt%.

Stream 8b is optional. In a variant of the invention, the stream 8 is entirely directed to unit E and the stream 8b is taken from said unit E, according to dotted line in Fig. 1. In further variants, stream 8b can be directed to other uses than concentration in section 3.

Stream 13 is preferably of demineralised water. A treated condensate recovered elsewhere in the urea process may also be used.

Fig. 1 relates to a plant adapted to produce the SCR solution 14 together with the urea melt 9. In a plant specifically designed for the production of the SCR solution, the full stream 8 may be directed to the section 10, and the concentration section 3 may be absent.

Fig. 2 shows a preferred embodiment of the invention. The acqueous stream 8 or a portion thereof, such as the portion 8a, is fed to a first evaporation unit E₁, obtaining a vapour stream 102 containing NH₃ and water, and a concentrated solution 103. A portion 107 of said concentrated solution is fed to a second evaporation unit E₂, obtaining a vapour phase 112 and a further concentrated solution with a very low ammonia content, indicated as stream 11. Said stream 11 is fed to the mixing device 12. The remaining portion 114 of the concentrated solution from the first unit E₁ is made available for other use(s), for example forming the stream 8b shown in dotted line in Fig. 1. In other embodiments, the full stream 103 may be directed to the second unit E₂.

Vapour streams 102 and 112 may be joined, as shown, and directed e.g. to a vacuum package of the plant (not shown) This embodiment of the invention is particularly effective, by providing substantially two steps in series for concentration and free NH₃ removal, in the evaporation units E₁ and E₂ respectively.

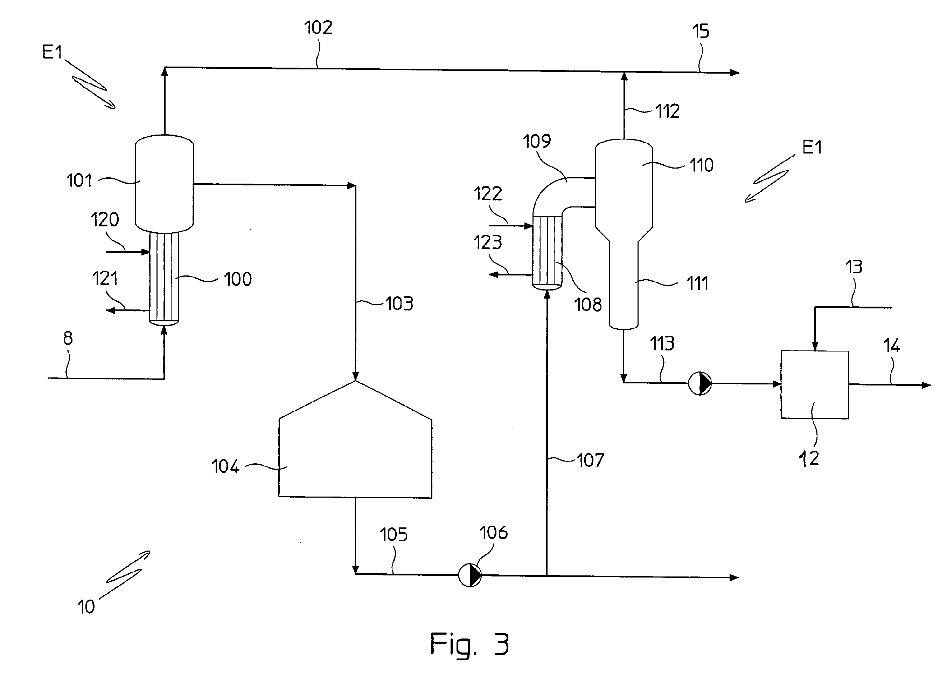

A particular embodiment of the invention is shown in greater detail in Fig. 3. The first unit E₁ comprises a steam-heated evaporator 100 and a flash vessel 101. In the example, the evaporator 100 is a shell-and-tube heat exchanger, where the urea-containing stream 8 is fed into the tubes, heated by steam 120 condensing on the shell side, and recovered as condensate 121.

The output of tube bundle of the evaporator 100 is passed into the flash vessel 101, where the vapour phase 102 containing ammonia and water is separated, obtaining a concentrated solution 103 which is stored in a tank 104. A stream 105 is taken from said tank and fed to a pump 106. Delivery of said pump is split into a stream 107 for the production of the SCR solution, and a stream 114 for other use(s) such as the production of urea melt in concentration section 3.

The stream 107 of concentrated solution is fed to the second evaporation unit E₂, comprising a heat exchanger 108 connected by a duct 109 to a vacuum separator 110. A vapour phase 112 containing water and urea is also separated from said separator 110, obtaining a further concentrated and substantially ammonia-free solution 113, sent to the mixer 12.

The second evaporation unit E₂ comprises a shell-and-tube heat exchanger where the stream 107 is fed into the tubes, heated by steam 122 fed to the shell side, and recovered as condensate 123.

A suitable separator is preferably installed in the vessel 101 to separate the liquid phase 103 from the vapours 102. The separator 110 has preferably a bottom well 111 for collecting the liquid phase forming the concentrated solution 113.

It should be noted that shell-and-tube heat exchangers are preferred for the exchanger(s) 100 and/or 108, but any conventional heat exchanger may be used.

An example is as follows. A stream 8 containing 68 wt% urea is evaporated in the evaporator 100 and passed in the flash tank 101 at 0.5 bar abs and 95 °C, obtaining a concentrated liquid stream 103 at 71 wt% urea. Stream 113 obtained in the second unit E₂ has 90 wt% urea and 430 ppm of free ammonia; said stream is then diluted with water 13 in the mixer 12, obtaining a stream 14 having 32 wt% urea and <200 ppm of ammonia. This stream 14 is adapted for use as SCR solution, i.e. for the removal of NOx in a SCR process. A cooler for the solution may be installed downstream the mixing device 12.

Referring again to Fig. 1, a conventional plant for the production of urea, comprising the synthesis section 1, the recovery section 2 and the concentration section 3, can be modified in order to use at least part of the output of the section 2 to produce the SCR solution 14. The modification involves the provision of at least the section 10 and related piping and accessories, such as pumps, valves, etc... The section 10 in turn may comprise the units E₁ and E₂ as in Fig. 2-3 and as described above. The modified urea plant, hence, is able to produce the SCR solution 14.

## Claims

1. A process for the preparation of an acqueous solution of urea suitable for use in a SCR process for nitrogen oxides removal, wherein an acqueous stream (8, 8a) containing urea and obtained in the recovery section (2) of a urea production plant is used for preparing said solution, the process being **characterized in that**:
- said acqueous stream (8, 8a) is subject to at least one process step of evaporation, separating a vapour stream (15) containing water and ammonia, and obtaining a concentrated and substantially ammonia-free solution (11);
- said concentrated solution (11) is then diluted to obtain a solution (14) with a concentration of urea suitable for use in the SCR process.

2. A process according to claim 1, wherein the evaporation comprises a heating step and a subsequent separation under vacuum, where the vapour stream containing water and ammonia and the concentrated solution are separated.

3. A process according to claim 2, wherein said separation under vacuum is carried out at a pressure of 0.2 to 0.4 bar.

4. A process according to any of claims 1 to 3, wherein said acqueous stream (8) from the recovery section (2) is subject to a first evaporation step, obtaining a vapour phase (102) containing water and ammonia and a concentrated urea solution (103) in liquid phase; at least a portion (107) of said concentrated urea solution is then subject to a second evaporation step, separating a vapour phase (112) containing water and ammonia and obtaining a further concentrated and substantially ammonia-free urea solution (11); said further concentrated urea solution (11) is then diluted with water (13) to a predetermined concentration of urea.

5. A process according to any one of the previous claims, wherein evaporation is carried out in shell-and-tube indirect heat exchanger(s), where the urea solution is fed to the tube side.

6. A process according to any one of the previous claims, wherein concentration of the solution after the dilution with water is 30 to 35 wt% urea.

7. A process according to any one of the previous claims, wherein the NH₃ content of the diluted solution is less than 2000 ppm and preferably less than 200 ppm.

8. A plant for the production of acqueous solution of urea suitable for use in a SCR process for nitrogen oxides removal, the plant comprising at least: a synthesis section (1) converting ammonia and carbon dioxide into a first acqueous stream (6) containing urea, ammonium carbamate and unreacted free ammonia; a recovery section (2) adapted to dissociate the carbamate and recycle ammonia and carbon dioxide to the synthesis section, and producing a second acqueous stream (8) substantially containing urea, water and residual ammonia, the plant being **characterized by** comprising a further section (10) adapted to convert at least a part (8a) of said second stream (8) into a solution (14) with a lower urea concentration for use in a SCR process, said further section (10) comprising:
- at least one evaporation unit (E), adapted to obtain a concentrated and substantially ammonia-free urea solution (11),
- a mixing device (12) adapted to dilute said concentrated solution (11) with water.

9. A plant according to claim 8, wherein said further section (10) comprises at least a first evaporation unit (E1) and a second evaporation unit (E2) operating in series.

10. A plant according to claim 9, wherein said further section (10) comprises a first evaporation unit (E1) obtaining a vapour phase containing water and ammonia and a concentrated urea solution in liquid phase, and a second evaporation unit (E2), said second unit being fed with at least a portion of said concentrated urea solution from the first unit, and obtaining a further concentrated and substantially ammonia-free urea solution, to be diluted for the production of the solution for use in SCR process.

11. A plant according to claim 10, wherein the first evaporation unit (E1) comprises an evaporator and a flash vessel, and the second evaporation unit (E2) comprises an evaporator and a vacuum separator.

12. A method for modifying an existing urea production plant, to make it adapted to produce a urea solution suitable for use in a SCR process, the plant comprising a synthesis section (1), a recovery section (2) and a concentration section (3), the recovery section (2) obtaining an acqueous stream (8) substantially containing urea, water and residual ammonia, fed to the concentration section (3) for the production of high-purity urea, the method comprising the provision of at least a further section (10) adapted to convert at least a part (8a) of said acqueous stream (8) into a solution (14) with a lower concentration of urea, for use in a SCR process, said further section (10) comprising at least one evaporation unit (E), adapted to obtain a concentrated and substantially ammonia-free urea solution (11), and a mixing device (12) adapted to dilute said concentrated solution (11) with water.
